# EUROPÄISCHE PATENTSCHRIFT

(11) **EP 2 023 831 B1**
(45) Veröffentlichungstag und Bekanntmachung des Hinweises auf die Patenterteilung: **23.03.2011**
(21) Anmeldenummer: 07724807.8
(22) Anmeldetag: 03.05.2007
(51) Int. Cl.: A61B 17/34

(54) **CHIRURGISCHER OBTURATOR**
SURGICAL OBTURATOR
OBTURATEUR CHIRURGICAL

(30) Priorität: 27.05.2006 DE 102006024757
(43) Veröffentlichungstag der Anmeldung: 18.02.2009
(73) Patentinhaber: Aesculap AG, 78532 Tuttlingen (DE)
(72) Erfinder: MALIGLOWKA, Johann, 78600 Kolbingen (DE); MAYENBERGER, Rupert, 78239 Rielasingen (DE); SCHWEITZER, Tom, 78532 Tuttlingen (DE)
(74) Vertreter: Hoeger, Stellrecht & Partner Patentanwälte
(86) Internationale Anmeldenummer: PCT/EP2007/003881
(87) Internationale Veröffentlichungsnummer: WO 2007/137672

(56) Entgegenhaltungen:
- EP-A- 0 551 968
- EP-A2- 0 135 364
- DE-U1-202006 008 405
- US-A- 5 324 268
- US-A- 5 423 770
- US-A- 5 569 289
- US-A- 5 645 076
- US-A- 5 779 680
- US-A- 5 807 402

## Beschreibung

Die Erfindung betrifft einen chirurgischen Obturator zum Durchstechen einer Körperwand mit einem rohrförmigen Gehäuse, das an einem Ende eine einen stetig abnehmenden Durchmesser aufweisende Einführspitze ausbildet, und mit einem über die Einführspitze vorstehenden Messer zum Einbringen eines Schnittes in die Körperwand.

Derartige Obturatoren werden verwendet, um Öffnungen für eine Kanüle oder ein Rohr in einer Körperwand auszubilden, bei dem Rohr kann es sich beispielsweise um ein Trokarrohr handeln, durch welches hindurch Instrumente in den Körper eingeführt werden.

Bei Vorschieben des Obturators besteht die Gefahr, dass beim vollständigen Durchtritt durch die Körperwand der Obturator unkontrolliert weiter vorgeschoben wird und das am vorderen Ende des Obturators angeordnete Messer Verletzung an inneren Organen hervorruft.

Es ist daher bekannt, derartige Obturatoren mit einem Schutzschild zu versehen, welches nach dem Durchtritt durch die Körperwand nach vorne geschoben wird und das Messer abdeckt (WO89/03661). Das Vorschieben des Schutzschildes kann ebenfalls zu Verletzungen führen, da dieses häufig unter der Wirkung einer Feder ruckartig nach vorne schnellt, außerdem ist es üblicherweise notwendig, dass der Obturator die Körperwand vollständig durchdrungen hat, bis das Schutzschild auslösen kann.

Das Dokument US 5807402 A offenbart einen Obturator gemäss dem Oberbegriff von Anspruch 1.

Es ist Aufgabe der Erfindung, einen gattungsgemäßen chirurgischen Obturator so auszubilden, dass die Gefahr einer Verletzung innerer Organe beim Durchstechen einer Körperwand herabgesetzt wird.

Diese Aufgabe wird bei einem chirurgischen Obturator der eingangs beschriebenen Art erfindungsgemäß dadurch gelöst, dass die Einführspitze eine zentrale Schutzkappe umfasst, die relativ zu der Einführspitze in Längsrichtung verschieblich zwischen einer vorgeschobenen Ruhestellung und einer zurückgezogenen Arbeitsstellung verschiebbar ist, dass das Messer im Gehäuse in Längsrichtung zwischen einer vorgeschobenen Schneidstellung und einer zurückgezogenen Schutzstellung verschiebbar ist, dass im Gehäuse eine das Messer aus der Schneidstellung in die Schutzstellung verschiebende Rückzugeinrichtung angeordnet ist und dass die Rückzugeinrichtung durch eine Verschiebung der Schutzkappe von der Arbeitsstellung in die Ruhestellung aktivierbar ist.

Bei einer solchen Ausgestaltung erfolgt der Schutz durch ein aktives Zurückziehen des Messers in das Gehäuse. Diese Rückzugeinrichtung wird ausgelöst durch die Bewegung einer zentralen Schutzkappe, die in einer Arbeitsstellung zurückgezogen und in einer Ruhestellung vorgeschoben ist. Eine solche Schutzkappe wird durch die Anlage des Obturators an der Körperwand in die Arbeitsstellung verschoben und gelangt beim Durchstechen der Körperwand in die vorgeschobene Ruhestellung, da die Schutzkappe dann nicht mehr an der Körperwand anliegt und dadurch in die Arbeitsstellung zurückgeschoben wird. Diese Vorschubbewegung der Schutzkappe wird ausgenutzt, um sofort das Messer so weit zurückzuziehen, dass es nicht mehr über die Schutzkappe und die Einführspitze vorsteht, so dass auch keine Verletzungsgefahr mehr besteht.

Es ist dabei vorteilhaft, wenn die Schutzkappe von einer Feder beaufschlagt ist, welche die Schutzkappe von der Arbeitsstellung in die Ruhestellung verschiebt. Dies erfolgt unmittelbar nach dem Durchstechen der Körperwand, die Vorschubbewegung der Schutzkappe kann dabei sehr gering sein, so dass keine Gefahr besteht, dass die Schutzkappe durch die Vorschubbewegung eine Verletzung erzeugt.

Insbesondere ist vorgesehen, dass das Messer in der Schutzstellung so weit zurückgezogen ist, dass es auch nicht mehr über die Schutzkappe in deren Arbeitsstellung hervorsteht. Selbst wenn also die Schutzkappe an einem inneren Organ zur Anlage kommen sollte und dadurch erneut in die Arbeitsstellung verschoben werden sollte, besteht keine Gefahr, dass das Messer mit inneren Organen in Kontakt kommt. Bei Verwendung eines das Messer überdeckenden Schutzschildes besteht die Gefahr, dass dieses unbeabsichtigt zurückgeschoben werden kann und dann das Messer freigibt. Um das zu verhindern, müssen spezielle, komplizierte Verriegelungsvorrichtungen vorgesehen sein, die den Schutzschild in der vorgeschobenen Stellung halten. Bei der beschriebenen Konstruktion entfällt diese Problematik, da das Messer in einer zurückgezogenen, geschützten Stellung verbleibt, wenn einmal durch den Vorschub der Schutzkappe die Rückzugseinrichtung aktiviert ist.

In der Schneidstellung steht das Messer im Bereich der zurückgezogenen Schutzkappe und in einem sich anschließenden Bereich der Einführspitze über die Kontur der Schutzkappe und der Einführspitze hervor.

Es ist dabei vorteilhaft, wenn das Messer in der Schneidstellung nicht über die Kontur der Einführspitze in deren proximalem Endbereich hervorsteht. Eine solche Ausgestaltung führt dazu, dass beim Durchstechen der Körperwand ein Schnitt nur in einem zentralen Bereich der Einführspitze erfolgt, nicht jedoch im Bereich des proximalen Randes. Beim weiteren Vorschieben des Obturators tritt die sich im proximaler Richtung in ihrem Durchmesser vergrößernde Einführspitze durch die durch den Schnitt erzeugte Öffnung in die Körperwand ein und dehnt diese auf, ohne im Endbereich noch zu schneiden. Es ergibt sich dadurch eine relativ schonende Erzeugung einer Öffnung und außerdem eine gute Abdichtung zwischen der Körperwand und dem Obturator, so dass gegebenenfalls auch eine im wesentlichen gasdichte Abdichtung erreicht werden kann. Dies ist wesentlich für den Fall einer Insufflation des Körperinnenraumes und bei Verwendung von abgedichteten Obturatoren.

Insbesondere kann die Einführspitze kegelstumpfförmig ausgebildet sein und eine abgerundete Spitze aufweisen. Dabei wird der zentrale Teil der Einführspitze in der Regel durch die Schutzkappe gebildet.

Die Verwendung einer zentralen Schutzkappe zur Auslösung der Rückzugeinrichtung hat weiterhin den Vorteil, dass ein Rückzug des Messers bereits eingeleitet werden kann, wenn die Einführspitze noch nicht vollständig durch die Körperwand hindurchgetreten ist. Es genügt nämlich, dass die zentrale Schutzkappe, deren Außendurchmesser kleiner ist als der Außendurchmesser der Einführspitze, durch die Körperwand hindurchgetreten ist, bereits dann erfolgt ein Rückzug des Messers, obwohl die Einführspitze noch nicht vollständig durch die Körperwand hindurchgeschoben ist. Durch die Auswahl der Abmessungen der Schutzkappe relativ zur übrigen Einführspitze kann der Zeitpunkt festgelegt werden, an dem während des Durchstechens ein Rückzug des Messers erfolgt. Je kleiner der Durchmesser der Schutzkappe im Verhältnis zum Durchmesser der Einführspitze ist, desto früher erfolgt die Aktivierung der Rückzugeinrichtung.

Bei einer bevorzugten Ausführungsform ist das Messer an einem Messerträger gehalten, der im Gehäuse längsverschieblich gelagert ist und der durch eine Feder in eine zurückgezogene Stellung verschiebbar ist, in der das Messer in der Schutzstellung steht.

Der Messerträger kann ein Griffelement tragen zum Verschieben des Messerträgers in die vorgeschobene Stellung.

Es ist günstig, wenn die Rückzugeinrichtung eine Raste umfasst, die den Messerträger beim Vorschieben in der vorgeschobenen Stellung arretiert.

Bei einer solchen Ausgestaltung kann vorgesehen sein, dass die Schutzkappe beim Vorschieben aus der Arbeitsstellung in die Ruhestellung die Raste löst und damit die Rückbewegung des Messerträgers in die zurückgezogene Stellung auslöst.

Bei einer besonders bevorzugten Ausführungsform verschiebt die Schutzkappe in der Arbeitsstellung ein Blockierelement in eine die Raste in einer Raststellung fixierende Position und entfernt das Blockierelement beim Vorschieben in die Ruhestellung wieder aus dieser Position. Durch die Entfernung des Blockierelements wird die Raste freigegeben und ermöglicht dem Messerträger die Rückbewegung in die Schutzstellung des Messers.

Besonders günstig ist eine Ausgestaltung, bei welcher die Raste durch ein Sperrglied in einer die Raste in einer Raststellung fixierenden Position gehalten wird und bei welcher die Schutzkappe in der Arbeitsstellung das Sperrglied von der Raste entfernt und gleichzeitig das Blockierelement in die die Raste fixierende Position verschiebt, so dass die Raste dabei kontinuierlich in ihrer Raststellung verbleibt.

Eine solche Ausgestaltung ermöglicht eine Verriegelung der Raste in der Raststellung auch dann, wenn die Schutzkappe sich noch nicht in Arbeitsstellung befindet, also noch in die Ruhestellung vorgeschoben ist. Sobald die Schutzkappe beim Durchstechen der Körperwand an die Außenseite der Körperwand angelegt wird, wird sie dadurch in die Arbeitsstellung verschoben und ersetzt dabei das Sperrglied durch das Blockierelement, das heißt die Schutzkappe selbst übernimmt die Blockierfunktion der Raste. Damit ist die Raste zu Beginn des Arbeitsvorgangs durch das Sperrglied in der Raststellung festgelegt, solange die Schutzkappe noch in der vorgeschobenen Ruhestellung steht und der Obturator noch nicht an die Körperwand angelegt ist. Bei Beginn des Durchstechvorgangs wird die Schutzkappe durch die Anlage an der Körperwand zurückgeschoben und übernimmt die Fixierung der Raste in der Raststellung. Diese Fixierung dauert so lange, bis die Schutzkappe nach dem Durchstechen in die Ruhestellung vorgeschoben wird und dadurch die Raste freigibt. Dies führt zum Zurückziehen des Messers.

Das Sperrglied kann am Messerträger gelagert sein, wobei die Lagerung insbesondere eine verschiebliche Lagerung ist.

Beispielsweise kann das Sperrglied ein den Messerträger umgebender, zwischen zwei Endstellungen frei verschieblicher Schiebering sein.

Günstig ist es, wenn die Raste ein federbelasteter Schwenkhebel ist.

Die Raste und der Messerträger können Aufgleitflächen aufweisen, die bei nicht in der Raststellung fixierter Raste beim Zurückschieben des Messerträgers aneinander aufgleiten und dabei die Raste in eine Freigabestellung bewegen. Die Raste wird also dadurch gelöst, dass der Messerträger mit einer Kraft in Richtung auf die Schutzstellung des Messers beaufschlagt ist, beispielsweise durch eine Feder.

Die Form des Messers kann sehr unterschiedlich sein, beispielsweise V-förmig, besonders günstig ist ein Messer mit einer schraubenlinienförmigen Schneidkante. Eine derartige schraubenlinienförmige Schneidkante erleichtert das gefühlvolle Einschieben des Obturators in eine Körperwand und führt auch dazu, dass beim Zurückziehen des Messers dieses durch die Führung des Messers in einem Führungsschlitz der Schutzkappe schraubenförmig bewegt wird und damit entsprechend der Einstechbewegung, die der Operateur ebenfalls schraubenlinienförmig vornimmt.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht eines Obturators im Bereich der Ein- führspitze;
- Figur 2:: eine Draufsicht auf den Obturator der Figur 1;
- Figur 3:: eine Detailansicht eines Messers mit einer schraubenlinienförmi- gen Messerschneide;
- Figuren 4 bis 7:: verschiedene Ausführungsformen bevorzugter Konturen von Mes- serschneiden;
- Figur 8:: eine Teillängsschnittansicht durch ein Gehäuse eines Obturators mit einer Rückzugeinrichtung für das Messer in einem der zurück- gezogenen Schutzstellung des Messers entsprechenden Zustand;
- Figur 9:: eine Ansicht ähnlich Figur 8 beim Vorschieben des Messers von der Schutzstellung in die Schneidstellung;
- Figur 10:: eine Ansicht ähnlich Figur 9 bei vollständig vorgeschobenem Mes- ser;
- Figur 11:: eine Ansicht ähnlich Figur 1 mit einem Obturator vor dem Anset- zen an einer Körperwand und vor dem Verschieben der Schutz- kappe in die Arbeitsstellung;
- Figur 12:: eine Ansicht ähnlich Figur 10 mit vorgeschobenem und in der Schneidstellung verriegeltem Messerträger;
- Figur 13:: eine Ansicht ähnlich Figur 11 mit dem Obturator beim Durchste- chen einer Körperwand mit der Schutzkappe in Arbeitsstellung;
- Figur 14:: eine Ansicht ähnlich Figur 12 mit einem zurückgeschobenen Mes- serträger;
- Figur 15:: eine schematische Seitenansicht des Obturators der Figur 1 nach dem Durchstechen der Körperwand und nach dem Zurückziehen des Messers und
- Figur 16:: eine Ansicht ähnlich Figur 14 bei der Rückbewegung des Messer- trägers in die Schutzstellung des Messers.

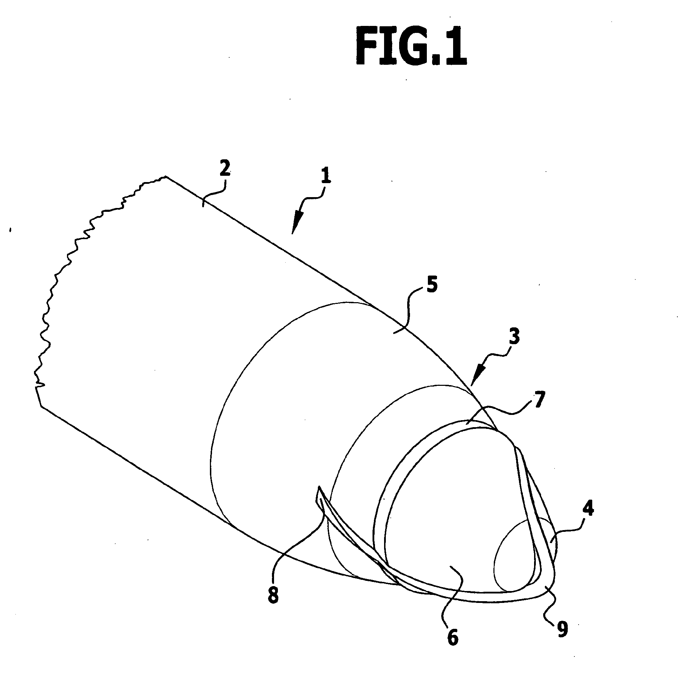

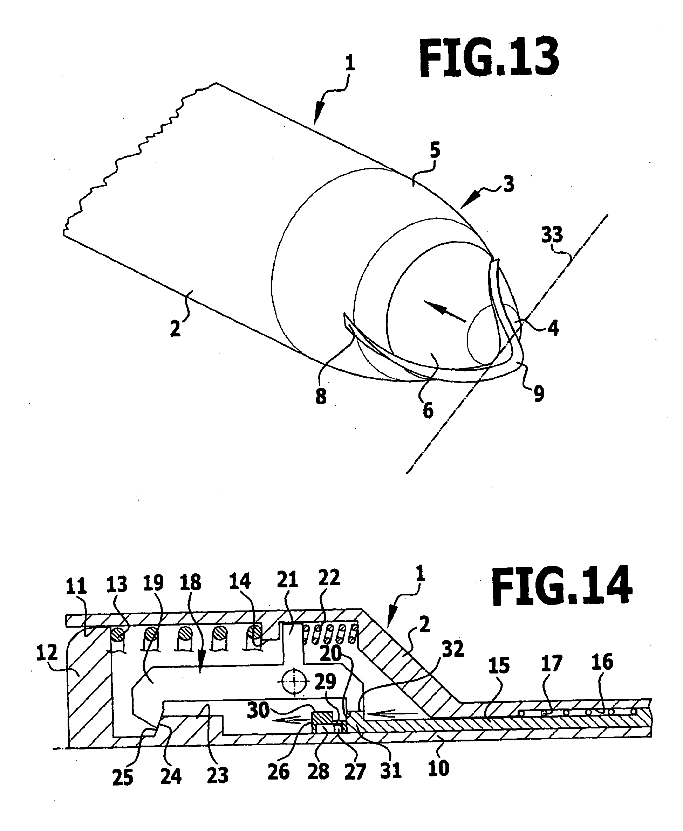

Der in der Zeichnung dargestellte Obturator 1 umfasst ein rohrförmiges Gehäuse 2, von dem in den Figuren 1, 11, 13 und 15 nur jeweils das vordere Teil dargestellt ist. Das Gehäuse 2 schließt in diesem vorderen Teil mit einer kegelstumpfförmigen Einführspitze 3 ab, deren Spitze 4 abgerundet ist. Die Einführspitze 3 ist in zwei Abschnitte unterteilt, nämlich einen proximalen Abschnitt 5, der Teil des Gehäuses 2 ist, und eine zentrale Schutzkappe 6, die gegenüber dem Gehäuse und damit gegenüber dem proximalen Abschnitt 5 in axialer Richtung zwischen zwei Stellungen verschiebbar ist, nämlich einer zurückgezogenen (proximalen) Arbeitsstellung und einer vorgeschobenen (distalen) Ruhestellung. In der zurückgezogenen Arbeitsstellung geht die Außenfläche der Schutzkappe 6 stetig in die Außenfläche des proximalen Abschnitts 5 über (Figur 13), in der vorgeschobenen Ruhestellung bildet sich zwischen der Schutzkappe 6 und dem proximalen Abschnitt 5 der Einführspitze 3 eine Stufe 7 aus (Figur 1).

Die Schutzkappe 6 weist einen durch ihre Spitze 5 hindurchlaufenden Schlitz 8 auf, der sich bis in den proximalen Abschnitt 5 der Einführspitze 3 erstreckt, der jedoch im Bereich dieses proximalen Abschnitts 5 endet, sich also nicht bis zum proximalen Ende des proximalen Abschnitts 5 erstreckt.

Durch diesen Schlitz 8 ragt ein Messer 9 hindurch, dessen Schneide in dem dargestellten Ausführungsbeispiel schraubenlinienförmig ausgebildet ist, dieser Verlauf entspricht auch dem Verlauf des Schlitzes 8. Die Schneide des Messers 9 verläuft dabei in einer vorgeschobenen Schneidstellung des Messers 9 in geringem Abstand von der Schutzkappe 6 und dem proximalen Abschnitt 5 ausserhalb dieser Teile, und das Messer 9 kann in eine zurückgezogene Schutzstellung verschoben werden, in der das Messer 9 nicht mehr aus dem Schlitz 8 hervorragt, und zwar auch dann nicht, wenn die Schutzkappe 6 in die zurückgezogene Arbeitsstellung verschoben ist.

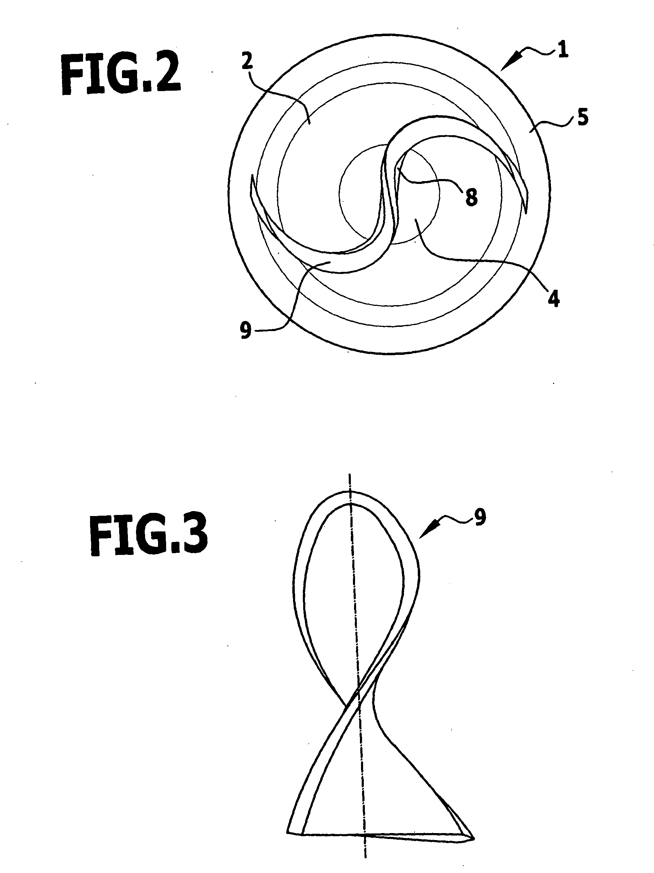

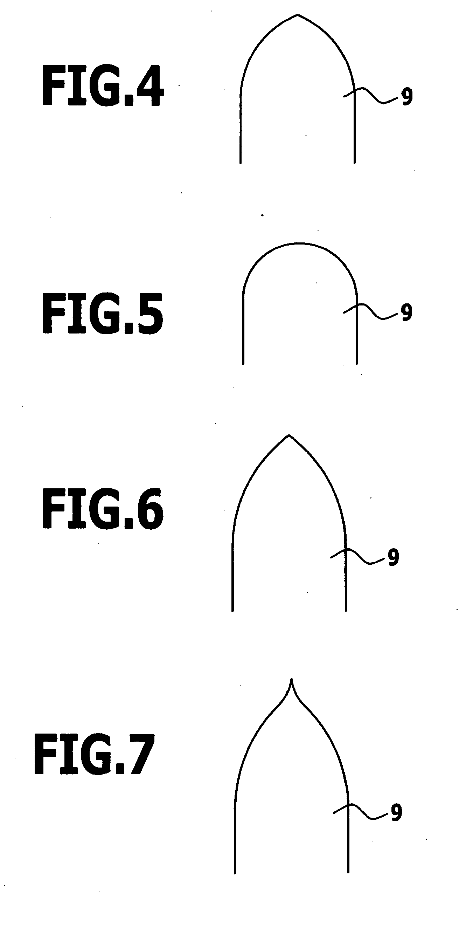

Die Kontur des Messers 9 kann sehr verschieden sein, neben der schraubenlinienförmigen Ausgestaltung gemäß Figuren 1 bis 3 können auch V-förmige Konturen oder ähnliche Konturen verwendet werden, wesentlich ist lediglich, dass die Schneide in Längsrichtung des Obturators 1 über die Einführspitze 3 vorsteht und dass die Messerschneide über die Spitze 4 verläuft, so dass beim Vorschieben des Obturators gegen eine Körperwand ausgehend von der Spitze ein ziehender Schnitt in die Körperwand eingebracht wird. In den Figuren 4 bis 7 sind mögliche Konturen der Messer 9 dargestellt, beispielsweise mit flachem oder steilem Spitzbogen (Figuren 4 und 6) mit halbkreisförmiger Kontur oder mit einer expliziten Ausbildung einer Spitze (Figur 7).

Das Messer 9 ist im Inneren des Gehäuses 2 des Obturators 1 an einem Messerträger 10 gehalten, der gegebenenfalls auch einstückig mit dem Messer 9 ausgebildet sein könnte, dieser Messerträger 10 erstreckt sich durch das gesamte Gehäuse 2 und verschließt eine Öffnung 11 an dem der Einführspitze 3 gegenüberliegenden Ende des Gehäuses 2 in Form einer Druckplatte 12. Im Inneren des Gehäuses 2 ist eine an deren Innenwand anliegende, den Messerträger 10 konzentrisch umgebende Schraubenfeder 13 angeordnet, die sich einerseits an der Druckplatte 12 und andererseits an einer Ringschulter 14 des Gehäuses 2 abstützt und die die Druckplatte 12 und damit den Messerträger 10 sowie das Messer 9 in eine zurückgezogene Stellung verschiebt, die der Schutzstellung des Messers entspricht. Gegen die Wirkung der Schraubenfeder 13 kann durch einen Druck auf die Druckplatte 12 die Einheit aus Messerträger 10 und Messer 9 in Richtung auf die Einführspitze 3 verschoben werden, so dass das Messer 9 in die Schneidstellung gelangt, in der die Messerschneide durch den Schlitz 8 nach außen vorsteht.

Die Schutzkappe 6 ist verbunden mit einer den Messerträger 10 umgebenden Hülse 15, die auch der Führung der Schutzkappe 6 dient und diese damit längsverschieblich in dem Obturator 1 lagert. In einem Ringspalt 16 zwischen der Innenwand des Gehäuses 2 und der Hülse 15 ist eine Schraubenfeder 17 angeordnet, die sich einerseits an der Innenwand des Gehäuses 2 und andererseits an der Hülse 15 abstützt und die Hülse 15 und damit die Schutzkappe 6 in die ausgeschobene Stellung verschiebt, die der Ruhestellung der Schutzkappe 6 entspricht. Gegen die Wirkung der Schraubenfeder 17 kann die Schutzkappe 6 in das Gehäuse 2 eingeschoben werden und gelangt dabei in die Arbeitsstellung.

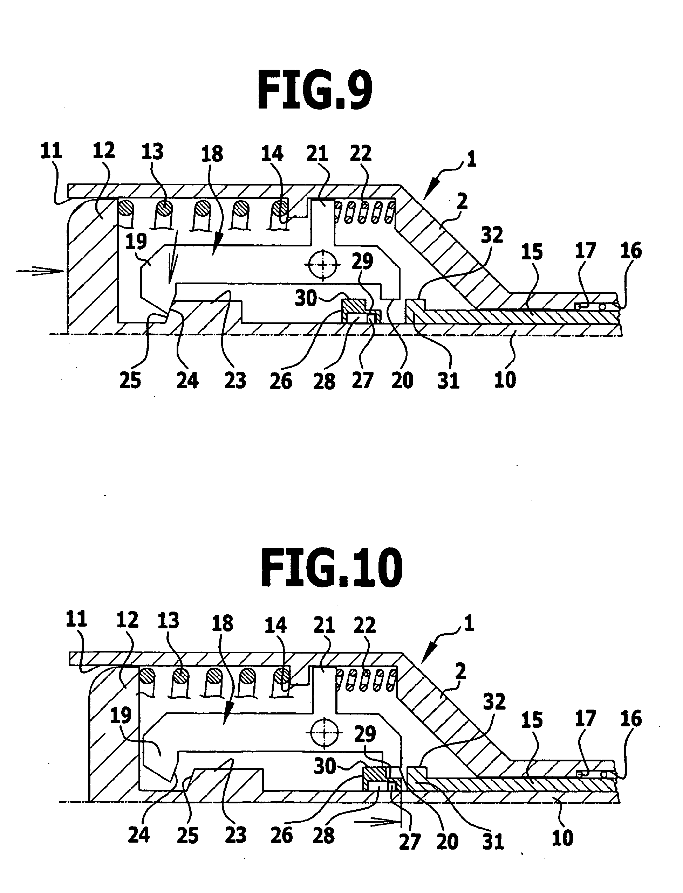

Im Gehäuse 2 ist weiterhin um eine quer zur Längsachse verlaufende Schwenkachse verschwenkbar ein Rasthebel 18 gelagert, der an einem Ende einen Rastvorsprung 19 und am gegenüberliegenden Ende eine Sperrfläche 20 aufweist. Durch eine einerseits an der Innenwand des Gehäuses 2 und andererseits an einem seitlichen Vorsprung 21 abgestützte Schraubenfeder 22 wird der Rasthebel 18 in eine Ausgangslage verschwenkt, in der der Rasthebel 18 parallel zur Längsrichtung verläuft. Dabei liegt der Vorsprung 21 an der Ringschulter 14 an (Figur 9).

Der Messerträger 10 weist einen seitlichen Rastvorsprung 23 auf, der radial so weit vorsteht, dass der Rastvorsprung 19 des Rasthebels 18 in die Bewegungsbahn des Rastvorsprungs 23 eintaucht, wenn der Messerträger 10 verschoben wird. Sowohl der Rastvorsprung 19 als auch der Rastvorsprung 23 weisen abgeschrägte Aufgleitflächen 24, 25 auf, die aneinander anliegen, wenn der Messerträger 10 von der vorgeschobenen Stellung in die zurückgezogene Stellung zurückgezogen wird.

Der Messerträger 10 wird umgeben von einem auf ihm längsverschieblich gelagerten Schiebering 26, dessen Längsverschiebung auf dem Messerträger 10 durch einen radial vom Messerträger 10 abstehenden Stift 27 begrenzt wird, der in eine quer zur Ebene des Schieberings 26 verlaufende Innennut 28 des Schieberings 26 eintaucht. Der Schiebering 26 liegt in aus der Zeichnung nicht ersichtlicher Weise an der Innenwand des Gehäuses 2 an und ist gegenüber dieser Innenwand verschiebbar. Zur Verschiebung ist jedoch eine gewisse Kraft nötig, um die Reibung zwischen Schiebering und Innenwand zu überwinden. Dies führt dazu, dass der Schiebering bei einer Verschiebung des Messerträgers 10 und damit des Stiftes 27 von der Bewegung des Messerträgers nur dann mitgenommen wird, wenn der Stift 27 am Ende der Innennut 28 anschlägt.

Der Schiebering 26 weist an seinem der Einführspitze 3 zugewandten Ende eine stufenförmige Ausnehmung 29 auf, die danebenliegende Außenfläche 30 ist in derselben Ebene angeordnet wie eine am Ende der Hülse 15 durch einen Ringflansch 31 gebildete Außenfläche 32 dieses Ringflansches.

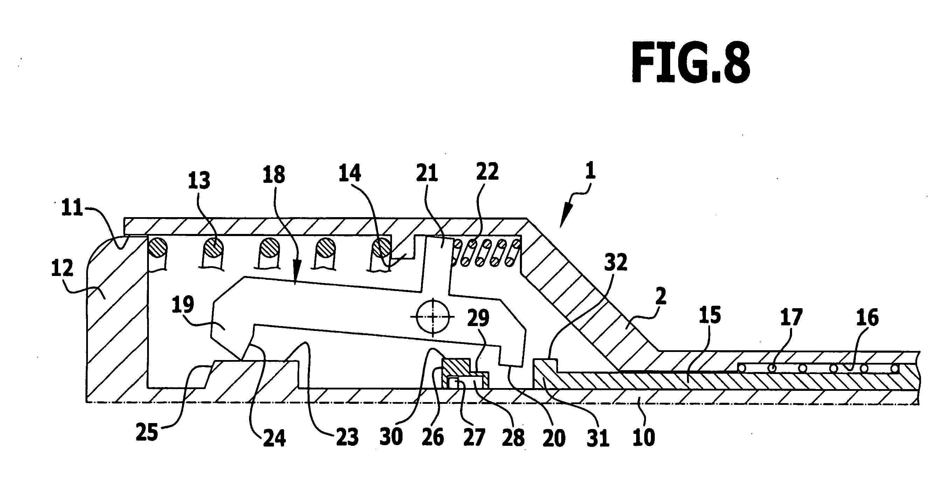

Bei der Benutzung des Obturators 1 steht zunächst der Messerträger 10 in der zurückgezogenen Stellung, in der er durch die Schraubenfeder 13 verschoben wird, das Messer 9 befindet sich daher in der zurückgezogenen Schutzstellung. In der zurückgezogenen Stellung des Messerträgers 10 stützt sich der Rastvorsprung 19 des Rasthebels 18 auf dem Rastvorsprung 23 ab, so dass der Rasthebel 18 gegen die Wirkung der Schraubenfeder 22 aus der Ausgangslage heraus verschwenkt ist (Figur 8). Die Sperrfläche 20 des Rasthebels 18 taucht dabei in einen Zwischenraum zwischen dem Schiebering 26 und dem Ringflansch 31 ein. Die Schutzkappe 6 und die Hülse 15 sind durch die Schraubenfeder 17 in die vorgeschobene Stellung verschoben, das heißt die Schutzkappe 6 befindet sich in ihrer Ruhestellung. In dieser Stellung der Schutzkappe 6 und des Messer 9 deckt die Schutzkappe 6 das Messer ab, das Messer steht nicht über den Schlitz 8 hervor. Die Schutzkappe 6 kann nicht in die zurückgezogene Arbeitsstellung verschoben werden, dies wird durch den zwischen den Schiebering 26 und den Ringflansch 31 eintauchenden Rasthebel 18 verhindert.

Zum Einsatz des Obturators 1 muss dieser aktiviert werden. Dies erfolgt durch einen Druck auf die Druckplatte 12, so dass diese entgegen der Wirkung der Schraubenfeder 13 verschoben wird. Dabei gleitet zunächst der Rastvorsprung 19 vom Rastvorsprung 23 herunter, so dass der Rasthebel 18 unter der Wirkung der Schraubenfeder 22 wieder in die Ausgangslage zurückschwenken kann (Figur 9).

Beim weiteren Vorschieben der Druckplatte 12 gelangt der Schiebering 26 mit seiner Außenfläche 30 unter die Sperrfläche 20 des Rasthebels 18 (Figur 10).

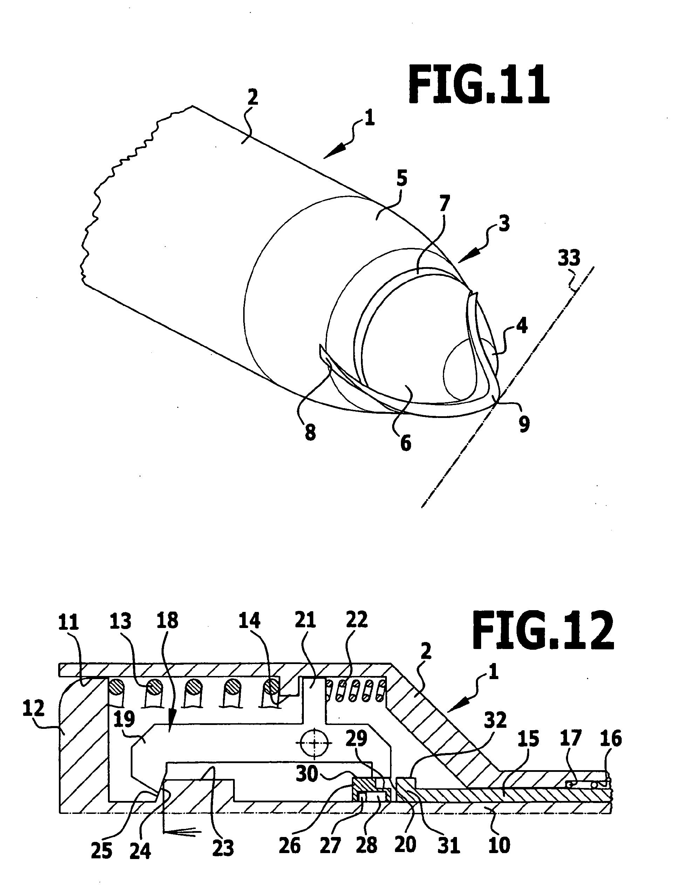

Lässt der Benutzer daraufhin die Druckplatte los, verschiebt sich der Messerträger 10 unter der Wirkung der Schraubenfeder 13 wieder ein wenig in die zurückgezogene Stellung zurück, dabei gelangt jedoch der Rastvorsprung 23 zur Anlage an dem Rastvorsprung 19, der eine weitere Rückbewegung des Messerträgers 10 verhindert. Der Rasthebel 18 liegt weiterhin an der Außenfläche 30 des Schieberings 26 an, der durch die Klemmwirkung zwischen Schiebering 26 und Sperrfläche 20 bei dieser Rückbewegung des Messerträgers 10 relativ zum Messerträger 10 verschoben wird, er bleibt relativ zur Sperrfläche 20 in seiner Position konstant. Durch diese Anlage der Außenfläche 30 des Schieberings 26 an der Sperrfläche 20 des Rasthebels 18 ist dieser daran gehindert, aus der Ausgangslage herauszuschwenken und blockiert daher die vollständige Rückbewegung des Messerträgers 10 in die zurückgezogene Lage (Figur 12). Durch die schrägen Aufgleitflächen 24 und 25 und unter der Wirkung der Schraubenfeder 13, die den Messerträger 10 in die zurückgezogene Stellung verschieben will, erfährt der Rasthebel 18 ein Drehmoment, welches seine Sperrfläche 20 kräftig gegen die Außenfläche 30 des Schieberings 26 drückt, so dass sichergestellt ist, dass diese Blockierung aufrechterhalten bleibt (Figur 12).

In dieser Stellung ist das Messer 9 in die Schneidstellung vorgeschoben, wird aber noch von der Schutzkappe 6 überfangen, die sich ihrerseits unter der Wirkung der Schraubenfeder 17 in der vorgeschobenen Ruhestellung befindet (Figuren 11 und 12).

Beim Vorschieben des Obturators 1 gegen eine Körperwand 33 legt sich die Spitze der Schutzkappe 6 gegen die Körperwand 33 und wird dadurch entgegen der Wirkung der Feder 17 zurückgeschoben, so dass das Messer 9, das in der Schneidstellung steht, freigegeben wird und nunmehr beim weiteren Vorschieben des Obturators 1 die Körperwand mit einem Schnitt durchsticht. Die Schutzkappe 6 bleibt dabei in der Anlage an der Körperwand 33 und wird somit dauerhaft in die Arbeitsstellung zurückgeschoben. Diese Rückbewegung führt auch dazu, dass die Hülse 15 mit ihrem Ringflansch 31 den Schiebering 26 gegenüber dem Messerträger 10 zurückschiebt, dabei tritt gleichzeitig der Ringflansch 31 unter die Sperrfläche 20 des Rasthebels 18 und übernimmt somit die Festlegung des Rasthebels 18 in der axialen Ausgangslage. Der Rasthebel 18 bleibt also in der Raststellung verriegelt, dabei erfolgt die Verriegelung am Anfang der Rückbewegung der Hülse 15 durch den Schiebering 26 und am Ende durch den Ringflansch 31 (Figur 14).

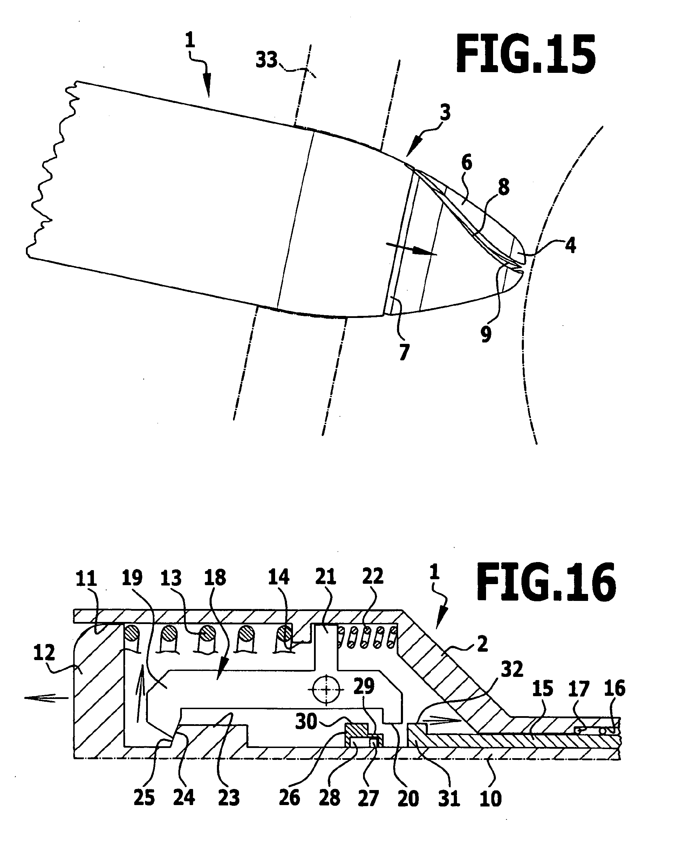

Sobald der Obturator 1 so weit durch die Körperwand 33 vorgeschoben ist, dass die Schutzkappe 6 innerhalb der Körperwand 33 angeordnet ist (Figur 15) kann die Schutzkappe 6 unter der Wirkung der Schraubenfeder 17 nach vorne in die Ruhestellung verschoben werden, da sie nicht mehr von der Körperwand 33 zurückgeschoben wird. Diese Vorschubbewegung der Schutzkappe 6 in die Ruhestellung führt auch dazu, dass der Ringflansch 31 unter der Sperrfläche 20 weggezogen wird, das heißt die Verriegelung des Rasthebels 18 wird aufgehoben, dieser kann aus der achsparallelen Ausgangslage gegen die Wirkung der Feder 22 verschwenkt werden.

Eine derartige Verschwenkung erfolgt durch die Rückschiebebewegung des Messerträgers 10 unter der Wirkung der Schraubenfeder 13. Bei dieser Rückbewegung gleiten die Aufgleitflächen 24, 25 der beiden Rastvorsprünge 19 und 23 aneinander auf, so dass der Rastvorsprung 23 am verschwenkten Rasthebel 18 und dessen Rastvorsprung 19 vorbeigleiten kann in die zurückgezogene Stellung, in der das Messer in der Schutzstellung steht.

Durch das Vorschieben der Schutzkappe 6 in die Ruheposition wird somit eine Rückschiebebewegung des Messers 9 in die Schutzstellung ausgelöst, so dass das Messer 9 nicht mehr über den Schlitz 8 vorsteht, die Gefahr einer Verletzung besteht damit nicht mehr, sobald die Schutzkappe 6 durch die Körperwand 33 hindurchgetreten ist.

Dieser Zeitpunkt ist erreicht, bevor die Einführspitze 3 vollständig die Körperwand 33 durchdrungen hat, da der Außendurchmesser der Schutzkappe 6 kleiner ist als der Außendurchmesser der Einführspitze 3. Beim vollständigen Hindurchschieben der Einführspitze 3 in das Körperinnere wird die von dem Messer 9 erzeugte Öffnung aufgeweitet, ohne dass dabei ein weiterer Schnitt erfolgt, da das Messer 9 dabei bereits in der Schutzstellung steht.

## Patentansprüche

1. Chirurgischer Obturator (1) zum Durchstechen einer Körperwand mit einem rohrförmigen Gehäuse (2), das an einem Ende eine einen stetig abnehmenden Durchmesser aufweisende Einführspitze (3) ausbildet, mit einem über die Einführspitze (3) vorstehenden Messer (9) zum Einbringen eines Schnittes in die Körperwand und mit einem Schutzelement (6), das relativ zum Gehäuse in Längsrichtung verschieblich zwischen einer vorgeschobenen Ruhestellung und einer zurückgezogenen Arbeitsstellung verschiebbar ist, wobei das Messer (9) im Gehäuse (2) in Längsrichtung zwischen einer vorgeschobenen Schneidstellung und einer zurückgezogenen Schutzstellung verschiebbar ist, wobei im Gehäuse (2) eine das Messer (9) aus der Schneidstellung in die Schutzstellung verschiebende Rückzugseinrichtung (13, 18) angeordnet ist und wobei die Rückzugeinrichtung (13, 18) durch eine Verschiebung des Schutzelementes (6) von der Arbeitsstellung in die Ruhestellung aktivierbar ist, wobei weiterhin die Einführspitze (3) in zwei Abschnitte (5, 6) unterteilt ist, nämlich einen proximalen Abschnitt (5), der Teil des Gehäuses (2) ist, und eine das Schutzelement ausbildende zentrale Schutzkappe (6), wobei die Außenfläche der Schutzkappe (6) in der zurückgezogenen Arbeitsstellung stetig in die Außenfläche des proximalen Abschnittes (5) übergeht, in der vorgeschobenen Ruhestellung sich dagegen zwischen der Schutzkappe (6) und dem proximalen Abschnitt (5) der Einführspitze (3) eine Stufe (7) ausbildet, und wobei das Messer (9) in der Schneidstellung im Bereich der zurückgezogenen Schutzkappe (6) und in einem sich anschließenden Bereich des proximalen Abschnittes (5) über die Kontur der Schutzkappe (6) und des Abschnittes (5) hervorsteht, **dadurch gekennzeichnet, dass** die Einführspitze (3) kegelstumpfförmig ausgebildet ist und eine abgerundete Spitze (4) aufweist und dass die Schutzkappe (6) einen durch ihre Spitze hindurch laufenden Schlitz (8) aufweist, der sich bis in den proximalen Abschnitt (5) der Einführspitze (3) erstreckt, der jedoch im Bereich dieses proximalen Abschnitts (5) endet, sich also nicht bis zum proximalen Ende des proximalen Abschnitts (5) erstreckt, und durch den das Messer (9) in der Schneidstellung hindurchragt.

2. Chirurgischer Obturator nach Anspruch 1, **dadurch gekennzeichnet, dass** das Messer (9) in der Schutzstellung so weit zurückgezogene ist, dass es auch nicht mehr über die Schutzkappe (6) in deren Arbeitsstellung hervorsteht.

3. Chirurgischer Obturator nach Anspruch 1 oder 2, **dadurch gekennzeichnet, dass** die Schutzkappe (6) von einer Feder (17) beaufschlagt ist, welche die Schutzkappe (6) von der Arbeitsstellung in die Ruhestellung verschiebt.

4. Chirurgischer Obturator nach einem der voranstehenden Ansprüche, **dadurch gekennzeichnet, dass** das Messer (9) in der Schneidstellung nicht über die Kontur des proximalen Abschnittes (5) der Einführspitze (3) in dessen proximalem Endbereich hervorsteht.

5. Chirurgischer Obturator nach einem der voranstehenden Ansprüche, **dadurch gekennzeichnet, dass** das Messer (9) an einem Messerträger (10) gehalten ist, der im Gehäuse (2) längsverschieblich gelagert ist und der durch eine Feder (13) in eine zurückgezogene Stellung verschiebbar ist, in der das Messer (9) in der Schutzstellung steht.

6. Chirurgischer Obturator nach Anspruch 5, **dadurch gekennzeichnet, dass** der Messerträger (10) ein Griffelement (12) trägt zum Verschieben des Messerträgers (10) in die vorgeschobene Stellung.

7. Chirurgischer Obturator nach Anspruch 5 oder 6, **dadurch gekennzeichnet, dass** die Rückzugeinrichtung eine Raste (18) umfasst, die den Messerträger (10) beim Vorschieben in der vorgeschobenen Stellung arretiert.

8. Chirurgischer Obturator nach Anspruch 7, **dadurch gekennzeichnet, dass** die Schutzkappe (6) beim Vorschieben aus der Arbeitsstellung in die Ruhestellung die Raste (18) löst und damit die Rückbewegung des Messerträgers (10) in die zurückgezogene Stellung auslöst.

9. Chirurgischer Obturator nach Anspruch 8, **dadurch gekennzeichnet, dass** die Schutzkappe (6) in der Arbeitsstellung ein Blockierelement (31) in eine die Raste (18) in einer Raststellung fixierende Position verschiebt und das Blockierelement (31) beim Vorschieben in die Ruhestellung wieder aus dieser Position entfernt.

10. Chirurgischer Obturator nach Anspruch 9, **dadurch gekennzeichnet, dass** die Raste (18) durch ein Sperrglied (26) in einer die Raste (18) in einer Raststellung fixierenden Position gehalten wird und dass die Schutzkappe (6) in der Arbeitsstellung das Sperrglied (26) von der Raste (18) entfernt und gleichzeitig das Blockierelement (31) in die die Raste (18) fixierende Position verschiebt, so dass die Raste (18) dabei kontinuierlich in ihrer Raststellung verbleibt.

11. Chirurgischer Obturator nach Anspruch 10, **dadurch gekennzeichnet, dass** das Sperrglied (26) am Messerträger (10) gelagert ist.

12. Chirurgischer Obturator nach Anspruch 11, **dadurch gekennzeichnet, dass** das Sperrglied (26) am Messerträger (10) verschieblich gelagert ist.

13. Chirurgischer Obturator nach Anspruch 12, **dadurch gekennzeichnet, dass** das Sperrglied (26) ein den Messerträger (10) umgebender, zwischen zwei Endstellungen frei verschieblicher Schiebering ist.

14. Chirurgischer Obturator nach einem der Ansprüche 7 bis 13, **dadurch gekennzeichnet, dass** die Raste (18) ein federbelasteter Schwenkhebel ist.

15. Chirurgischer Obturator nach einem der Ansprüche 7 bis 14, **dadurch gekennzeichnet, dass** die Raste (18) und der Messerträger (10) Aufgleitflächen (24, 25) aufweisen, die bei nicht in der Raststellung fixierter Raste (18) und beim Zurückschieben des Messerträgers (10) aneinander aufgleiten und dabei die Raste (18) in eine Freigabestellung bewegen.

16. Chirurgischer Obturator nach einem der voranstehenden Ansprüche, **dadurch gekennzeichnet, dass** das Messer (9) eine schraubenlinienförmige Schneidkante aufweist.

## Claims

1. Surgical obturator (1) for piercing a body wall, comprising a tubular housing (2) that at one end forms an introduction tip (3) with a continuously decreasing diameter, comprising a blade (9) projecting from the introduction tip (3) for making an incision in the body wall, and comprising a protective element (6), which is displaceable in longitudinal direction relative to the housing between an advanced inoperative position and a retracted working position, wherein the blade (9) in the housing (2) is displaceable in longitudinal direction between an advanced cutting position and a retracted protected position, wherein disposed in the housing (2) is a retraction device (13, 18) that displaces the blade (9) from the cutting position into the protected position, and wherein the retraction device (13, 18) may be activated by a displacement of the protective element (6) from the working position into the inoperative position, wherein, furthermore, the introduction tip (3) is subdivided into two portions (5, 6), namely a proximal portion (5), which is part of the housing (2), and a central protective cap (6) forming the protective element, wherein the outer surface of the protective cap (6) in the retracted working position verges continuously into the outer surface of the proximal portion (5), whereas in the advanced inoperative position a step (7) is formed between the protective cap (6) and the proximal portion (5) of the introduction tip (3), and wherein the blade (9) in the cutting position projects in the region of the retracted protective cap (6) and in an adjoining region of the proximal portion (5) from the contour of the protective cap (6) and of the portion (5), **characterized in that** the introduction tip (3) is of a truncated-cone-shaped design and has a rounded tip (4) and **in that** the protective cap (6) has a slot (8) running through its tip, which slot (8) extends as far as into the proximal portion (5) of the introduction tip (3) but terminates in the region of this proximal portion (5), i.e. does not extend as far as the proximal end of the proximal portion (5), and through which slot (8) the blade (9) projects in the cutting position.

2. Surgical obturator according to claim 1, **characterized in that** the blade (9) in the protected position is retracted to such an extent that it also no longer projects from the protective cap (6) in the working position thereof.

3. Surgical obturator according to claim 1 or 2, **characterized in that** the protective cap (6) is acted upon by a spring (17) that displaces the protective cap (6) from the working position into the inoperative position.

4. Surgical obturator according to one of the preceding claims, **characterized in that** the blade (9) in the cutting position does not project beyond the contour of the proximal portion (5) of the introduction tip (3) in the proximal end region thereof.

5. Surgical obturator according to one of the preceding claims, **characterized in that** the blade (9) is held on a blade carrier (10), which is mounted in a longitudinally displaceable manner in the housing (2) and which is displaceable by means of a spring (13) into a retracted position, in which the blade (9) is in the protected position.

6. Surgical obturator according to claim 5, **characterized in that** the blade carrier (10) carries a gripping element (12) for displacing the blade carrier (10) into the advanced position.

7. Surgical obturator according to claim 5 or 6, **characterized in that** the retraction device comprises a catch (18), which during advancing locks the blade carrier (10) in the advanced position.

8. Surgical obturator according to claim 7, **characterized in that** the protective cap (6) during advancing from the working position into the inoperative position releases the catch (18) and hence triggers the return movement of the blade carrier (10) into the retracted position.

9. Surgical obturator according to claim 8, **characterized in that** the protective cap (6) in the working position displaces a locking element (31) into a position fixing the catch (18) in a detent position and during advancing into the inoperative position removes the locking element (31) from this position.

10. Surgical obturator according to claim 9, **characterized in that** the catch (18) is held by means of a blocking member (26) in a position fixing the catch (18) in a detent position and that the protective cap (6) in the working position removes the blocking member (26) from the catch (18) and at the same time displaces the locking element (31) into the position fixing the catch (18), in which case the catch (18) remains continuously in its detent position.

11. Surgical obturator according to claim 10, **characterized in that** the blocking member (26) is mounted on the blade carrier (10).

12. Surgical obturator according to claim 11, **characterized in that** the blocking member (26) is mounted displaceably on the blade carrier (10).

13. Surgical obturator according to claim 12, **characterized in that** the blocking member (26) is a sliding ring that surrounds the blade carrier (10) and is freely displaceable between two end positions.

14. Surgical obturator according to one of claims 7 to 13, **characterized in that** the catch (18) is a spring-loaded pivoted lever.

15. Surgical obturator according to one of claims 7 to 14, **characterized in that** the catch (18) and the blade carrier (10) have sliding faces (24, 25), which, when the catch (18) is not fixed in the detent position and the blade carrier (10) is being pushed back, slide along one another and in so doing move the catch (18) into a release position.

16. Surgical obturator according to one of the preceding claims, **characterized in that** the blade (9) has a helical-line-shaped cutting edge.

## Revendications

1. Obturateur chirurgical (1) pour transpercer ou perforer une paroi corporelle, comprenant un boitier (2) de forme tubulaire, qui forme, à une extrémité, une pointe d'introduction (3) présentant un diamètre diminuant en continu, comprenant également une lame coupante (9) qui fait saillie de la pointe d'introduction (3) pour la réalisation d'une incision dans la paroi corporelle, ainsi qu'un élément de protection (6) qui peut coulisser dans la direction longitudinale par rapport au boitier, entre une position de repos avancée et une position de travail retirée vers l'arrière, obturateur chirurgical
dans lequel la lame coupante (9) peut coulisser dans le boitier (2) en direction longitudinale, entre une position de coupe avancée et une position de protection retirée vers l'arrière,
dans lequel dans le boitier (2) est agencé un dispositif de retrait vers l'arrière (13, 18), qui fait coulisser la lame coupante (9) de la position de coupe dans la position de protection, et le dispositif de retrait vers l'arrière (13, 18) peut être activé par un coulissement de l'élément de protection (6) de la position de travail à la position de repos,
dans lequel la pointe d'introduction (3) est par ailleurs subdivisée en deux tronçons (5, 6), à savoir un tronçon proximal (5), qui fait partie du boitier (2), et un cabochon de protection (6) central formant l'élément de protection,
dans lequel la surface extérieure du cabochon de protection (6), dans la position de travail retirée vers l'arrière, se raccorde de manière continue à la surface extérieure du tronçon proximal (5), mais, dans la position de repos avancée, un gradin (7) se forme par contre entre le cabochon de protection (6) et le tronçon proximal (5) de la pointe d'introduction (3), et
dans lequel la lame coupante (9), dans la position de coupe, fait saillie hors du contour du cabochon de protection (6) et du tronçon (5), dans la zone du cabochon de protection (6) retiré vers l'arrière et dans une zone du tronçon proximal (5) s'y raccordant,
**caractérisé en ce que** la pointe d'introduction (3) est d'une configuration de forme tronconique et présente une pointe (4) arrondie, et **en ce que** le cabochon de protection (6) présente une fente (8) passant par sa pointe et qui s'étend jusque dans le tronçon proximal (5) de la pointe d'introduction (3), mais se termine toutefois dans la zone de ce tronçon proximal (5) et ne s'étend donc pas jusqu'à l'extrémité proximale du tronçon proximal (5), et à travers laquelle fait saillie la lame coupante (9) dans la position de coupe.

2. Obturateur chirurgical selon la revendication 1, **caractérisé en ce que** la lame coupante (9), dans la position de protection, est retirée vers l'arrière d'une valeur telle qu'elle ne fasse plus non plus saillie pardessus le cabochon de protection (6) dans sa position de travail.

3. Obturateur chirurgical selon la revendication 1 ou la revendication 2, **caractérisé en ce que** le cabochon de protection (6) est sollicité par un ressort (17), qui fait coulisser le cabochon de protection (6) de la position de travail dans la position de repos.

4. Obturateur chirurgical selon l'une des revendications précédentes, **caractérisé en ce que** la lame coupante (9), dans la position de coupe, ne fait pas saillie hors du contour du tronçon proximal (5) de la pointe
d'introduction (3) dans sa zone d'extrémité proximale.

5. Obturateur chirurgical selon l'une des revendications précédentes, **caractérisé en ce que** la lame coupante (9) est maintenue sur un support de lame (10) qui est monté en coulissement longitudinal dans le boitier (2), et peut coulisser, grâce à un ressort (13), dans une position retirée vers l'arrière, dans laquelle la lame coupante (9) se trouve dans la position de protection.

6. Obturateur chirurgical selon la revendication 5, **caractérisé en ce que** le support de lame (10) porte un élément de préhension (12) pour faire coulisser le support de lame (10) dans la position avancée.

7. Obturateur chirurgical selon la revendication 5 ou la revendication 6, **caractérisé en ce que** le dispositif de retrait vers l'arrière comprend un élément d'encliquetage (18), qui, lors de l'avancement, arrête le support de lame (10) dans la position avancée.

8. Obturateur chirurgical selon la revendication 7, **caractérisé en ce que** le cabochon de protection (6), lors de l'avancement de la position de travail vers la position de repos, débloque l'élément d'encliquetage (18), et déclenche ainsi le mouvement de retour du support de lame (10) dans la position retirée vers l'arrière.

9. Obturateur chirurgical selon la revendication 8, **caractérisé en ce que** le cabochon de protection (6), dans la position de travail fait coulisser un élément de blocage (31) dans une position fixant l'élément d'encliquetage (18) dans une position encliquetée, et éloigne à nouveau l'élément de blocage (31) de cette position lors de l'avancement dans la position de repos.

10. Obturateur chirurgical selon la revendication 9, **caractérisé en ce que** l'élément d'encliquetage (18) est maintenu par un organe de verrouillage (26) dans une position fixant l'élément d'encliquetage (18) dans une position encliquetée, et **en ce que** le cabochon de protection (6), dans la position de travail, éloigne l'organe de verrouillage (26) de l'élément d'encliquetage (18) et fait coulisser simultanément l'élément de blocage (31) dans la position fixant l'élément d'encliquetage (18), de sorte que l'élément d'encliquetage (18) reste à cette occasion continuellement dans sa position encliquetée.

11. Obturateur chirurgical selon la revendication 10, **caractérisé en ce que** l'organe de verrouillage (26) est monté sur le support de lame (10).

12. Obturateur chirurgical selon la revendication 11, **caractérisé en ce que** l'organe de verrouillage (26) est monté de manière coulissante sur le support de lame (10).

13. Obturateur chirurgical selon la revendication 12, **caractérisé en ce que** l'organe de verrouillage (26) est un anneau coulissant, qui entoure le support de lame (10) et peut coulisser librement entre deux positions extrêmes.

14. Obturateur chirurgical selon l'une des revendications 7 à 13, **caractérisé en ce que** l'élément d'encliquetage (18) est un levier pivotant chargé par ressort.

15. Obturateur chirurgical selon l'une des revendications 7 à 14, **caractérisé en ce que** l'élément d'encliquetage (18) et le support de lame (10) présentent des surfaces de rampe de glissement (24, 25) qui, pour un élément d'encliquetage (18) non fixé dans la position encliquetée et lors du retrait vers l'arrière du support de lame (10), glissent l'une sur l'autre et déplacent à cette occasion l'élément d'encliquetage (18) dans une position de dégagement.

16. Obturateur chirurgical selon l'une des revendications précédentes, **caractérisé en ce que** la lame coupante (9) présente un tranchant de coupe en forme d'hélice.
